(19) Europäisches Patentamt / European Patent Office / Office européen des brevets

(11) **EP 4 335 867 A1**

(12) **EUROPEAN PATENT APPLICATION**
published in accordance with Art. 153(4) EPC

(43) Date of publication:
**13.03.2024 Bulletin 2024/11**

(21) Application number: **22799104.9**

(22) Date of filing: **03.05.2022**

(51) International Patent Classification (IPC):
**C07K 14/47** (2006.01)  **C12N 15/70** (2006.01)

(52) Cooperative Patent Classification (CPC):
**C07K 14/47; C12N 15/70**

(86) International application number:
**PCT/KR2022/006356**

(87) International publication number:
**WO 2022/235061 (10.11.2022 Gazette 2022/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.05.2021 KR 20210057062**

(71) Applicant: **Cellemedy Co., Ltd
Gyeonggi-do 13605 (KR)**

(72) Inventors:
• **LEE, Jee Won
Seoul 06707 (KR)**

• **LEE, Bo Ram
Seongnam-si, Gyeonggi-do 13557 (KR)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

Remarks:
The complete document including Reference Table(s) and the Sequence Listing(s) can be downloaded from the EPO website

(54) **NOVEL PROTEIN**

(57) The present invention relates to a ferritin protein mutated to have a foreign peptide fused to the outer surface thereof and to decrease in binding affinity for human transferrin receptors, wherein the peptide can exhibit activities of various foreign peptides.

[FIG. 7]

EP 4 335 867 A1

**Description**

[Technical Field]

The present invention relates to a novel protein.

[Background Art]

The drug delivery system refers to a pharmaceutical technology capable of efficiently delivering a required amount of a drug, for example, protein, nucleic acid, or other small molecule substances by minimizing side effects of existing drugs while maximizing the efficacy and effects thereof. The above technology, which reduces the costs and time required for new drug development, has recently become a field of advanced technology that creates new added value in the pharmaceutical industry by combination with nanotechnology. Advanced countries in technology such as the U.S. and Japan have focused their efforts on the development of drug delivery system as well as new drug development centered around companies such as pharmaceutical companies since the later of 80s.
Till the present, viral genes, recombinant proteins, liposomes, cationic polymers, and various types of nanoparticles and nano materials have been used for drug delivery into animal cells. However, many cationic liposomes and cationic polymers were found to be unsuitable for clinical application due to their strong toxicity to cells. Further, a method of chemically modifying the main chain of a nucleic acid for stable nucleic acid penetration into a cell membrane has been tried. However, this method is not suitable for clinical application because it is expensive, takes a long time, and requires a labor-intensive process. As a meaningful attempt, a drug delivery system (DDS) using various types of nanoparticles including quantum dots, magnetic particles, or gold nanoparticles has been developed. However, these particles are toxic to cells, have a structure that is not easy for introduction of biopolymers such as nucleic acids, and have a disadvantage of low introduction efficiency to the cells.
An efficient delivery system is needed for the study of functions of proteins/peptides in the cells or for intracellular delivery. However, the development of a universal delivery system capable of delivering a wide range of proteins/peptides, and a system capable of receiving and delivering a large amount of proteins/peptides are still in an incomplete state.

[Summary of Invention]

[Problems to be Solved by Invention]

An object of the present invention is to provide a novel protein which includes a foreign peptide fused to an outer surface thereof, and is mutated so that a binding force to a human transferrin receptor is reduced.
Another object of the present invention is to provide a pharmaceutical composition including the above protein.

[Means for Solving Problems]

To achieve the above objects, the present invention provides a ferritin protein in which a foreign peptide is fused to an outer surface thereof, and a molecule capable of binding to an immune checkpoint molecule mutated so as to reduce a binding force to a human transferrin receptor is fused.
The foreign peptide may be a pharmacologically active peptide.
The foreign peptide may include a ligand or a fragment thereof, a receptor or a fragment thereof, an antibody or a fragment thereof including an antigen binding region (CDR), which are capable of binding to an immune checkpoint molecule; or a disease antigen epitope.
The immune checkpoint molecule may be any one selected from the group consisting of Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1 and NTRK2.
The antigen-binding site may be any one selected from the group consisting of HCDR1, HCDR2, HCDR3, LCDR1, LCDR2 and LCDR3.
The disease antigen epitope may be selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4, neoantigens and GV1001.

The protein of the present invention may be a spherical protein formed by self-assembly of 24 ferritin monomers to which the foreign peptide is fused.

In the present invention, the foreign peptide may be fused to at least one between adjacent α-helices of the ferritin monomer.

In the present invention, the foreign peptide may be fused to N-terminus or C-terminus of the ferritin monomer.

In the present invention, the foreign peptide may be fused to A-B loop, B-C loop, C-D loop or D-E loop of the ferritin monomer.

In the present invention, the foreign peptide may be fused between N-terminus and A helix or between E helix and C-terminus of the ferritin monomer.

The protein of the present invention may be characterized in that amino acid No. 15, 16, 23, 82, 84, 117, 120 or 124 in the sequence of SEQ ID NO: 1 is substituted with alanine, glycine, valine or leucine.

The binding force (K) of the ferritin protein of the present invention to the transferrin receptor may satisfy Equation 1 below:

$$[Equation\ 1]$$

$$K \geq 10\ nM$$

(in Equation 1, K is [P][T]/[PT], wherein [P] represents a concentration of ferritin protein in an equilibrium state of a binding reaction between the ferritin protein and the transferrin receptor, [T] represents a concentration of transferrin receptor in the equilibrium state, and [PT] represents a concentration of a complex of the ferritin protein and the transferrin receptor in the equilibrium state).

In the present invention, the binding force to the transferrin receptor may be a binding force to a human transferrin receptor.

In the present invention, the ferritin may be a human ferritin heavy chain.

The ferritin protein of the present invention may be present in an aqueous fraction of 40% or more in an E. coli production system.

[Advantageous effects]

The protein of the present invention has a reduced binding force to the human transferrin receptor, and thus can sufficiently exhibit activity derived from a foreign peptide.

The protein of the present invention may be fused with a plurality of foreign peptides, and thus can exhibit more enhanced activity compared to the foreign peptide itself.

The protein of the present invention has excellent biocompatibility and safety, and exhibits very low possibility of inducing an inflammatory response or immunogenicity.

[Brief Description of Drawings]

FIG. 1 is a schematic diagram of a basic vector for preparing wild type (native) human ferritin heavy chain (huHF) and mutant human ferritin heavy chain protein.

FIG. 2 is a schematic diagram of a vector for producing huHF_Mutant1-dual and a diagram confirming the production of a ferritin protein.

FIG. 3 is a schematic diagram of a vector for producing huHF_Mutant1-h_smPD1 and a diagram confirming the production of the ferritin protein.

FIGS. 4 to 6 illustrate results of evaluating the binding force of huHF_native-h_smPD1 and huHF_Mutant1-h_smPD1 to human transferrin receptor, respectively.

FIG. 7 illustrates results of evaluating the binding force of huHF_Mutant1-dual and huHF_Mutant2-dual to human transferrin receptor.

FIG. 8 illustrates results of evaluating the binding ability between NK cells and cancer cells according to huHF_mutant1-dual treatment.

FIGS. 9 and 10 illustrate results of evaluating the binding ability between NK cells and cancer cells according to huHF_mutant1-dual treatment.

FIG. 11 illustrates results of evaluating the cancer cell killing ability of NK cells according to huHF_mutant1-dual treatment.

FIG. 12 is a schematic diagram showing the preparation of an animal model of LLC1 lung cancer and tumor suppression experiment.

FIGS. 13 and 14 illustrate results of evaluating tumor growth inhibition according to huHF_mutant1-dual treatment.

FIGS. 15 to 17 illustrate results of evaluating avidity for the lung cancer cell line A549 of huHF_mutant1-dual.

FIG. 18 illustrates results of evaluating tumor growth inhibition in the animal model of LLC1 lung cancer when using huHF _mutant1-dual or in combination with an anticancer agent.

[Mode for Carrying out Invention]

The present invention relates to a ferritin protein in which a foreign peptide is fused to an outer surface thereof, and a molecule capable of binding to an immune checkpoint molecule mutated so as to reduce a binding force to a human transferrin receptor is fused.

The ferritin may be ferritin derived from humans, animals and microorganisms.

Human ferritin is composed of a heavy chain (21 kDa) and a light chain (19 kDa), and exhibits the feature of forming spherical nanoparticles through self-assembly ability of monomers constituting the ferritin. The ferritin may form a self-assembly having a spherical three-dimensional structure by gathering 24 monomers.

For the human ferritin, an outer diameter is about 12 nm and an inner diameter is about 8 nm. The structure of a ferritin monomer is a form in which five $\alpha$-helix structures, namely, A helix, B helix, C helix, D helix and E helix are sequentially connected, and may include an atypical polypeptide portion that links polypeptides having each $\alpha$-helix structure called a loop.

The loop is a region that is not structurally broken even when a peptide or a small protein antigen is inserted into the ferritin. Herein, the peptide is fused using cloning, such that a peptide-ferritin fusion protein monomer, in which a peptide such as an epitope is located in the ferritin monomer, may be prepared. The loop linking A helix and B helix is called A-B loop, the loop linking B helix and C helix is called B-C loop, the loop linking C helix and D helix is called C-D loop, and the loop linking D helix and E helix is called D-E loop.

Information on ferritin is known from the NCBI (GenBank Accession No. NM_000146, NM_002032 or the like).

The ferritin may be a ferritin heavy chain, and specifically, a human ferritin heavy chain. The human ferritin heavy chain may be used interchangeably with 'huHF' .

The ferritin protein may be a ferritin monomer. Accordingly, it may be a ferritin heavy chain monomer or a human ferritin heavy chain monomer.

Ferritin forms an organized structure or pattern by self-assembly of several monomers thereof. The ferritin protein of the present invention is nano-scaled particles. For example, 24 ferritin monomers fused with a molecule capable of binding to an immune checkpoint molecule may be self-assembled to form a ferritin protein.

The ferritin protein of the present invention may have a spherical shape. In the case of the spherical shape, for example, the particle diameter may be 8 to 50 nm. More specifically, it may be 8 to 50 nm, 8 to 45 nm, 8 to 40 nm, 8 to 35 nm, 8 to 30 nm, 8 to 25 nm, 8 to 20 nm, or 8 to 15 nm.

The ferritin protein of the present invention includes a foreign peptide fused to an outer surface thereof. The foreign peptide may be a foreign peptide to ferritin. It is sufficient that the foreign peptide is fused to the outer surface of the ferritin protein, and therefore, it is not necessary that the foreign peptide is fused to all ferritin monomers constituting the ferritin protein.

For example, when 24 ferritin monomers fused with a foreign peptide are self-assembled to form a ferritin protein, it is sufficient that the foreign peptide is fused to at least one ferritin monomer among 24 ferritin monomers. To increase the density of molecules capable of binding to immune checkpoint molecules on the outer surface of the ferritin protein, the foreign peptide can be fused to all 24 ferritin monomers.

One or two or more immune checkpoint molecules and foreign peptides may be fused to each ferritin monomer constituting the ferritin protein. Each ferritin monomer constituting the ferritin protein may be fused with different foreign peptides.

The foreign peptide may be a pharmacologically active peptide. Any peptide may be used without limitation as long as it has pharmacological activity, for example, a ligand or a fragment thereof, a receptor or a fragment thereof, an antibody or a fragment thereof including an antigen binding region (CDR), which are capable of binding to immune checkpoint molecules; or a disease antigen epitope.

Immune checkpoint molecules may bind to T cells and inactivate the same. Such immune checkpoint molecules may include, for example, Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1, NTRK2 and the like.

The molecule capable of binding to the immune checkpoint molecule may be a ligand or a fragment thereof, a receptor or a fragment thereof, an antibody or a fragment thereof including an antigen binding region (CDR), which are capable of binding to the immune checkpoint molecule.

The antibody may be any antibody having complementarity-determining regions (CDRs), and may include all antibodies belonging to classes IgA, IGD, IgE, IgG, IgM, IgY, IgW, etc., antigen-binding fragments thereof, scFvs and the like.

For example, the antibody may be an antibody against a disease antigen, an antibody against an immune checkpoint molecule, an antibody against an antigen to be detected for the purpose of diagnosis, prediction, evaluation, etc., or an antibody against an antigen that is a treatment target for a disease.

A disease antigen may be an antigen of any disease that can be prevented, treated, alleviated or ameliorated by an immune response. For example, the disease antigen may be a cell surface antigen of a cancer cell, a pathogen cell, or a cell infected with a pathogen. A particular site that determines the antigenic specificity of a disease antigen is the disease antigen epitope.

The disease includes all diseases that can be treated by the antibody, and may be, for example, an infectious disease, cancer, inflammatory disease and the like.

The infectious disease may be, for example, a viral, bacterial, fungal, parasitic or prion infection.

Inflammatory diseases may include, for example, atherosclerosis, arteriosclerosis, autoimmune disease, multiple sclerosis, systemic lupus erythematosus, polymyalgia rheumatica, gouty arthritis, degenerative arthritis, tendinitis, bursitis, psoriasis, cystic fibrosis, osteoarthritis, rheumatoid arthritis, inflammatory arthritis, Sjogren's syndrome, giant cell arteritis, progressive systemic sclerosis (scleroderma), ankylosing spondylitis, polymyositis, dermatomyositis, pemphigus, diabetes (e.g., type I), myasthenia gravis, Hashimoto's thyroiditis, Graves Disease, Goodpasture's disease, mixed connective tissue disease, sclerosing cholangitis, inflammatory bowel disease, Crohn's disease, ulcerative colitis, pernicious anemia, inflammatory dermatosis, common interstitial pneumonia, asbestosis, silicosis, bronchiectasis, berylliosis, talcosis, pneumoconiosis, sarcoidosis, dissecting interstitial pneumonia, lymphocytic interstitial pneumonia, giant cell interstitial pneumonia, cellular interstitial pneumonia, exogenous allergic alveolitis, Wegener's granulomatosis and related forms of vasculitis (temporal arteritis and nodular polyarteritis), inflammatory dermatitis, hepatitis, delayed-type hypersensitivity reactions (e.g., poison ivy), pneumonia, respiratory tract infections, adult respiratory distress syndrome, encephalitis, immediate hypersensitivity reaction, asthma, hay fever, allergies, acute anaphylaxis, rheumatic fever, Glomerulonephritis, pyelonephritis, cellulitis, cystitis, chronic cholecystitis, ischemia (ischemic injury), reperfusion injury, allograft rejection, host versus graft rejection, appendicitis, arteritis, blepharitis, bronchiolitis, bronchitis, cervicitis, cholangitis, chorioamnionitis, conjunctivitis, laryngitis, dermatomyositis, endocarditis, endometritis, enteritis, enterocolitis, epididymitis, epididymitis, fasciitis, fibrosis, gastritis, gastroenteritis, gingivitis, ileitis, iritis, laryngitis, myelitis, myocarditis, nephritis, umbilical corditis, ovarian, orchitis, osteitis, otitis, pancreatitis, parotitis, pericarditis, pharyngitis, pleurisy, phlebitis, pneumonia, proctitis, prostatitis, rhinitis, salpingitis, sinusitis, stomatitis, synovitis, orchitis, tonsillitis, urethritis, cystitis, uveitis, vaginitis, vasculitis, vulvitis, vulvovaginitis, vasculitis, chronic bronchitis, osteomyelitis, optic neuritis, temporal arteritis, transverse myelitis, necrotizing fasciitis and necrotizing enterocolitis.

Cancer may be a cancer selected from the group consisting of, for example, brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, stomach cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenocystic carcinoma, adenoma, adenomatous squamous carcinoma, anal duct cancer, anal cancer, anorectal cancer, astrocytoma, greater vestibular gland cancer, basal cell carcinoma, cholangiocarcinoma, bone cancer, bone marrow cancer, bronchial cancer, bronchial adenocarcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrioid adenocarcinoma, endothelial cell carcinoma, ependymal cell carcinoma, epithelial cell carcinoma, orbital cancer, focal nodular hyperplasia, gallbladder cancer, pyelonephroma, gastric basal cancer, gastrinoma, glioblastoma, glucagonoma, heart cancer, hemangioblastoma, hemangioendothelioma, hemangioma, hepato adenoma, liver cell adenoma, hepatobiliary tract cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colorectal cancer, lymphoma, malignant mesothelial cell tumor, medulloblastoma, medullary epithelioma, meningeal cancer, mesothelial cancer, metastatic carcinoma, oral cancer, mucoepidermoid carcinoma, multiple myeloma, muscle cancer, nasal duct cancer, nervous system cancer, non-epithelial skin cancer, Non-Hodgkin's lymphoma, soft cell carcinoma, oligodendroglioma cancer, mouth cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, Retinoblastoma, serous carcinoma, sinus cancer, skin cancer, small cell carcinoma, small intestine cancer, smooth muscle cancer, soft tissue cancer, somatostatin-secreting tumor, spinal cancer, epithelial squamous cell cancer, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma and Wilm's tumor.

The inflammatory disease antigen may be, for example, an inflammatory cytokine, and may include, for example: growth hormone such as human growth hormone, N-methionyl human growth hormone and bovine growth hormone; parathyroid hormone; thyroxine; insulin; proinsulin; relaxin; prorelaxin; glycoprotein hormones such as follicle stimulating hormone (FSH), thyroid stimulating hormone (TSH) and luteinizing hormone (LH); hepatic growth factor; fibroblast growth factor; prolactin; placental lactogen; tumor necrosis factor such as tumor necrosis factor-alpha (TNF-$\alpha$) and tumor necrosis factor-beta (TNF-$\beta$); mullerian inhibitory substances; mouse gonadotropin-associated peptide; inhibin; activin; vascular endothelial growth factor; integrin; human platelet production promoter (thrombopoietin, TPO); nerve growth factors such as NGF-alpha (NGF-$\alpha$); platelet-growth factor; placental growth factor; transforming growth factors (TGFs) such as TGF-$\alpha$ and TGF-$\beta$; insulin-like growth factor-1 and -11; erythropoietin (EPO); osteoinductive factors; interferons such as interferon-alpha (IFN-$\alpha$), interferon-beta (IFN-$\beta$) and interferon-gamma (IFN-$\gamma$); colony stimulating factors (CSFs) such as macrophage-CSF (M-CSF); granulocyte macrophage-CSF (GM-CSF); granulocyte-CSF (G-CSF); interleukins such as IL-1, IL-2, IL-3, IL-4, IL-5, IL-6, IL-7, IL-8, IL-9, IL-10, IL-11, IL-12, IL-13, IL-15, IL-17, IL-18, IL-21, IL-22, IL-23, IL-33, etc.; and other polypeptide factors including LIF and kit ligand (KL).

Cancer antigens may be, for example gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4, neoantigens or the like. The neoantigen refers to an immunogenic peptide induced and formed by somatic mutation in tumor cells. The neoantigen forms a complex with MHC I, moves to the surface of tumor cells and may be displayed as an antigenic epitope. T-cell receptor (TCR) may recognize this neoantigen-MHC I complex thus to induce an immune response.

The antibody may be, for example, an antibody against an immune checkpoint molecule on the surface of a cancer cell or T cell. For example, the antibody may be PD-1, Her-2/neu, VISTA, 4-1BBL, CD48, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD-L1, PD-L2, CD40L, LAG3, TIM3, TIGIT, BTLA, CD52, SLAMF7, 4-1BB, OX-40, ICOS, GITR, CD27, CD28, CD16, CD3, CD20, EGFR family, AXL, CSF1R, DDR1, DDR2, EPH receptor family, FGFR family, VEGFR family, IGF1R, LTK, PDGFR family, RET, KIT, KRAS, NTRK1 or NTRK2.

The antigen to be detected may be, for example, a biomarker. The biomarker may be, for example, a biomarker for diagnosing a disease, a biomarker for predicting the possibility of a disease, a biomarker for prognostic diagnosis of a specific drug, etc., but it is not limited thereto.

The CDR is a CDR as a component of an antibody, and may be, for example, at least one selected from the group consisting of HCDR1, HCDR2, HCDR3, LCDR1, LCDR2 and LCDR3, for example, HCDR3.

One or two or more CDRs may be fused to a ferritin monomer.

When two or more CDRs are fused, two or more same CDRs of the same antibody may be included, different CDRs of the same antibody may be included, or CDRs of different antibodies may be included.

In addition, the protein of the present invention may be self-assembled including a ferritin monomer fused with different CDRs.

The different CDRs may be different CDRs of the same antibody or CDRs of different antibodies.

Disease antigen epitopes may be selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4, neoantigen and GV1001.

The foreign peptide may be fused to any ferritin monomer as long as it can be exposed on the outer surface of the ferritin protein. The foreign peptide is fused to a position that does not interfere with self-assembly of the ferritin monomer.

Foreign peptides can be fused inside the ferritin monomer to change the structure of the ferritin protein. Among the respective components of the ferritin monomer, the internally indented part may protrude to an outside by fusion of the same with the foreign peptide. On the other hand, the portion protruding to the outside among the respective components of the ferritin monomer may be internally indented by fusion of the same with the foreign peptide.

The foreign peptide is preferably fused to a position where the ferritin protein reduces the binding force to the human transferrin receptor or interferes with the binding to the human transferrin receptor.

The foreign peptide is not limited to a specific range in terms of the structure, molecular weight, and amino acid length thereof.

The foreign peptide may be, for example, a ligand, antibody or fragment thereof whose amino acid length is 25aa or less. More specifically, the amino acid length may be 25aa or less, 24aa or less, 23aa or less, 22aa or less, 21aa or less, 20aa or less, 19aa or less, 18aa or less, 17aa or less, 16aa or less, 15aa or less, 14aa or less, 13aa or less, 12aa or less, 11aa or less or less, 10aa or less, 9aa or less, 8aa or less, 7aa or less, 6aa or less, 5aa or less. Further, for example, the amino acid may have a length of 3aa or more, 4aa or more, 5aa or more, 6aa or more, 7aa or more, 8aa or more, 9aa or more, 10aa or more.

The fusion site of the foreign peptide is not limited to a specific position, for example, the fusion may be performed inside the α-helix (A helix, B helix, C helix, D helix, or E helix), between adjacent α-helices, N-terminus, C-terminus, A-B loop, B-C loop, C-D loop, D-E loop, between N-terminus and A helix, between E helix and C-terminus, inside helix, etc. of the ferritin monomer.

The ferritin protein of the present invention is mutated so that it does not bind well to the transferrin receptor. For example, the ferritin protein of the present invention satisfies the following Equation 1 for the transferrin receptor:

$$[\text{Equation 1}]$$

$$K \geq 10 \text{ nM}$$

(in Equation 1, K is [P][T]/[PT], wherein [P] represents a concentration of ferritin protein in an equilibrium state of a binding reaction between the ferritin protein and the transferrin receptor, [T] represents a concentration of transferrin receptor in the equilibrium state, and [PT] represents a concentration of a complex of the ferritin protein and the transferrin receptor in the equilibrium state).

The binding force may be, for example, a binding force to the human transferrin receptor.

K value of Equation 1 is 10 nM or more, 20 nM or more, 30 nM or more, 40 nM or more, 50 nM or more, 60 nM or more, 70 nM or more, 80 nM or more, 90 nM or more, 100 nM or more, 110 nM or more, 120 nM or more, 125 nM or more, more than 125 nM, 150 nM or more, 200 nM or more, 210 nM or more, 220 nM or more, 230 nM or more, 240 nM or more, 250 nM or more, 260 nM or more, 270 nM or more, 280 nM or more, 290 nM or more, 300 nM or more, 350 nM or more, 400 nM or more, 450 nM or more, 500 nM or more, 550 nM or more, 600 nM or more, 700 nM or more, 800 nM or more, 900 nM or more, or 1000 nM or more. The higher the value of K in Equation 1, the lower the binding force to the transferrin receptor.

The binding force (K) to the transferrin receptor is measured in the equilibrium state of the binding reaction between the ferritin protein (A) of the present invention and the transferrin receptor. The concentration of the ferritin protein ([P]), the concentration of the transferrin receptor ([T]), and the concentration of the complex of the protein of the present invention and the transferrin receptor ([PT]) in the equilibrium state can be measured by various known methods.

The ferritin protein of the present invention may fuse the foreign peptide to a site involved in binding to the transferrin receptor in order to decrease the binding force to the transferrin receptor. For example, this protein may fuse the foreign peptide to be located at the B-C loop and the A helix portion of the ferritin protein of the present invention.

The ferritin protein of the present invention may be produced in a microorganism expressing a sequence encoding the protein.

Microorganisms known in the art may be used without limitation thereof. For example, it may be *E. coli,* and specifically BL21 (DE3), but it is not limited thereto.

In the case of producing a protein by a microbial system, it is easy to perform separation/purification only when the obtained protein is present in a dissolved state in the cytoplasm. In many cases, the prepared protein is present in an aggregated state as an inclusion body or the like. The ferritin protein of the present invention has a high rate of lysis in the cytoplasm in a microbial production system. Therefore, it is easy to perform separation/purification and utilize the same.

The ferritin protein of the present invention may be prepared of, for example, in an *E. coli* system for producing the same in a state in which the water-soluble fraction ratio of the total protein is 40% or more. Specifically, the fraction ratio may be 40% or more, 50% or more, 60% or more, 70% or more, 80% or more, 90% or more, or 95% or more. The upper limit thereof may be, for example, 100%, 99%, 98%, 97%, 96% or the like.

According to the present invention, the above ferritin protein may be utilized for various purposes depending on the type of the foreign peptide.

For example, when the foreign peptide is a pharmacologically active peptide, it may be utilized as a pharmaceutical composition applying such pharmacological activity. Specifically, in the case where the foreign peptide is a ligand or a fragment thereof, a receptor or a fragment thereof, an antibody or a fragment thereof including an antigen binding region (CDR), which are capable of binding to an immune checkpoint molecule; alternatively, a disease antigen epitope, the pharmaceutical composition may be intended to prevent or treat cancer, infectious disease, inflammatory disease and the like.

The present invention provides a pharmaceutical composition including the above ferritin protein. All of the above descriptions regarding the ferritin protein will be applied as they are to the ferritin protein as an active ingredient of the pharmaceutical composition of the present application.

The pharmaceutical composition of the present invention may include a pharmaceutically acceptable carrier. As used herein, the term "pharmaceutically acceptable carrier" refers to a carrier or diluent that does not inhibit the biological activity and properties of the administered component without significantly stimulating the organism. The pharmaceutically acceptable carrier in the present invention may be one component or a mixture of one or more components among saline, sterile water, Ringer's solution, buffered saline, dextrose solution, maltodextrin solution, glycerol and ethanol. Further, as necessary, other conventional additives such as antioxidants, buffers and bacteriostatic agents may be added to formulate an injection suitable for injection into tissues or organs. Furthermore, it may be formulated as an isotonic sterile solution, or in some cases, a dry preparation (especially a freeze-dried preparation) that can be an injectable solution by adding sterile water or physiological saline. In addition, a target organ-specific antibody or other ligand may be used in combination with the carrier so as to act specifically on the target organ.

Further, the composition of the present invention may further include a filler, an excipient, a disintegrant, a binder or a lubricant. Furthermore, the composition of the present invention may be formulated using methods known in the art to provide rapid, sustained or delayed release of the active ingredient after administration to a mammal.

In one embodiment, the pharmaceutical composition may be an injection formulation, and may be administered intravenously, but it is not limited thereto.

As used herein, the term "effective amount" refers to an amount necessary to delay the onset or progression of a specific disease to be treated or to promote it entirely.

In the present invention, the composition may be administered in a pharmaceutically effective amount. It is apparent to those skilled in the art that a suitable total daily amount of the pharmaceutical composition may be determined by a treating physician within the scope of sound medical judgment.

For the purposes of the present invention, a specific pharmaceutically effective amount for a specific patient is preferably applied differently depending on various factors including: type and extent of the response to be achieved; whether or not other agents are used if necessary; specific composition; patient's age, weight general health status, sex, diet; administration time; administration route; secretion rate of the composition; treatment period; drug used together or concurrently with the specific composition, as well as similar factors well known in the pharmaceutical field.

In the present invention, the pharmaceutical composition may be accompanied by an instruction associated with packaging in a form instructed by a government agency in charge of the manufacture, use and sale of drugs if necessary. The instruction indicates the approval of a private interest organization in regard to the form of a composition or administration to human or animals, and may be, for example, a label approved by the US Food and Drug Administration for the prescription of the drugs.

Cancer may be a cancer selected from the group consisting of, for example, brain cancer, head and neck cancer, bladder cancer, breast cancer, cervical cancer, colon cancer, colorectal cancer, endometrial cancer, esophageal cancer, leukemia, lung cancer, liver cancer, ovarian cancer, pancreatic cancer, prostate cancer, rectal cancer, kidney cancer, stomach cancer, testicular cancer, uterine cancer, vascular tumor, squamous cell carcinoma, adenocarcinoma, small cell carcinoma, melanoma, glioma, neuroblastoma, sarcoma, laryngeal cancer, parotid cancer, biliary tract cancer, thyroid cancer, actinic keratosis, acute lymphocytic leukemia, acute myeloid leukemia, adenocystic carcinoma, adenoma, adenomatous squamous carcinoma, anal duct cancer, anal cancer, anorectal cancer, astrocytoma, greater vestibular gland cancer, basal cell carcinoma, cholangiocarcinoma, bone cancer, bone marrow cancer, bronchial cancer, bronchial adenocarcinoma, carcinoid, cholangiocarcinoma, chronic lymphocytic leukemia, chronic myelogenous leukemia, clear cell carcinoma, connective tissue cancer, cystic adenoma, digestive system cancer, duodenal cancer, endocrine system cancer, endoderm sinus tumor, endometrial hyperplasia, endometrioid adenocarcinoma, endothelial cell carcinoma, ependymal cell carcinoma, epithelial cell carcinoma, orbital cancer, focal nodular hyperplasia, gallbladder cancer, pyelonephroma, gastric basal cancer, gastrinoma, glioblastoma, glucagonoma, heart cancer, hemangioblastoma, hemangioendothelioma, hemangioma, hepato adenoma, liver cell adenoma, hepatobiliary tract cancer, hepatocellular carcinoma, Hodgkin's disease, ileal cancer, insulinoma, intraepithelial neoplasia, intraepithelial squamous cell neoplasia, intrahepatic biliary tract cancer, invasive squamous cell carcinoma, jejunum cancer, joint cancer, pelvic cancer, giant cell carcinoma, colorectal cancer, lymphoma, malignant mesothelial cell tumor, medulloblastoma, medullary epithelioma, meningeal cancer, mesothelial cancer, metastatic carcinoma, oral cancer, mucoepidermoid carcinoma, multiple myeloma, muscle cancer, nasal duct cancer, nervous system cancer, non-epithelial skin cancer, Non-Hodgkin's lymphoma, soft cell carcinoma, oligodendroglioma cancer, mouth cancer, osteosarcoma, papillary serous adenocarcinoma, penile cancer, pharyngeal cancer, pituitary tumor, plasmacytoma, pseudosarcoma, pulmonary blastoma, rectal cancer, renal cell carcinoma, respiratory system cancer, Retinoblastoma, serous carcinoma, sinus cancer, skin cancer, small cell carcinoma, small intestine cancer, smooth muscle cancer, soft tissue cancer, somatostatin-secreting tumor, spinal cancer, epithelial squamous cell cancer, striatal muscle cancer, submesothelial carcinoma, T cell leukemia, tongue cancer, ureter cancer, urethral cancer, cervical cancer, uterine trunk cancer, vaginal cancer, VIPoma, vulvar cancer, highly differentiated carcinoma and Wilm's tumor.

The infectious disease may be, for example, a viral, bacterial, fungal, parasitic or prion infection.

Hereinafter, the present invention will be described in detail with reference to the following examples.

## EXAMPLE

### 1. Construction of expression vector for production of protein

huHF is spherical protein nanoparticles (12 nm) composed of 24 monomers, each of which is composed of a total of 5 $\alpha$-helices. The present inventors induce mutation in the amino acid sequence that binds to a transferrin receptor of the huHF monomer through gene cloning, such that huHF_Mutant1 (Q15A, D16A, R23A, F82A, Q84A), huHF_Mutant2 (Q15A, D16A, R23A, F82A, Q84A, E117A, K120A, D124A) were obtained as shown in Table 1 below. The antibody CDR3 peptide and domain were inserted into a selected position among the loop between $\alpha$-helices of huHF_Mutant1 and 2 (BC loop in huHF 5T to 176G based on the PDB 3AJO sequence; 92D/93W) and the C-terminus through gene cloning, so as to ensure the delivery system.

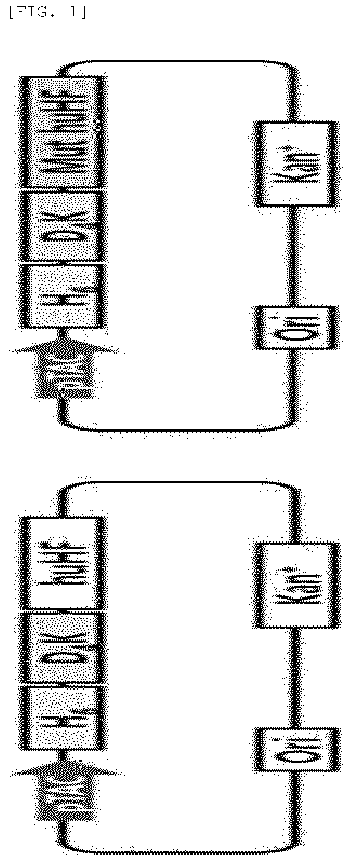

The sequence of Table 2 below was used, and PCR was performed according to the vector schematic diagram of FIGS. 1 to 3, and Table 3, thereby preparing huHF_Mutantl_1_dual (BC loops, C-terminus), huHF_Mutant2-dual (BC loops, C-terminus) and huHF_Mutant1-h_smPD1 (C-terminus). All the constructed plasmid expression vectors were purified on an agarose gel, and then the sequence was identified through complete DNA sequencing.

[TABLE 1]

| Designation | SEQ ID NO. |
|---|---|
| huHF_Native | 1 |
| huHF-Mutant1 | 2 |
| huHF-Mutant2 | 3 |

[TABLE 2]

| Designation | SEQ ID NO. |
|---|---|
| huHF-exPD-L1 | 4 |
| huHF $\alpha$TIGIT | 5 |
| huHF-h_smPD1 | 6 |
| Linker1 | 7 |
| Linker2 | 8 |
| Linker3 | 9 |

[TABLE 3]

| Protein | Expression vector |
|---|---|
| huHF Native | NH2-NdeI-huHF-$\alpha$PD-L1 HCDR3-HindIII-COOH |
| huHF_Mutant1-dual | BC(92D/93W): NH2-NdeI-huHF_Mutant1-[$\alpha$PD-L1 HCDR3]- huHF_Mutant1- $\alpha$TIGIT HCDR3-HindIII-COOH |
| huHF_Mutant2-dual | BC(92D/93W): NH2-NdeI-huHF_Mutant2-[$\alpha$PD-L1 HCDR3]-huHF- $\alpha$TIGIT HCDR3-HindIII-COOH |
| huHF_Mutant1-h smPD1 | NH2-NdeI-huHF_Mutant1-Linker2-h_smPD1-HindIII-COOH |

**2. Protein biosynthesis**

*E. coli* strain BL21 [(fhuA2 [lon] ompT gal [dcm] ΔhsdS)] was transformed with the expression vector prepared above, respectively, and a kanamycin-resistant transformant was selected. Transformed *E. coli* was cultured in a flask (250 mL Erlenmeyer flask, 37 °C, 150 rpm) including 50 mL of Luria-Bertani (LB) medium (containing 100 mg L-1 kanamycin). When the medium turbidity (O.D 600) reached about 0.5-0.7, Isopropyl-β-Dthiogalactopyanosid (IPTG) (1.0 mM) was injected to induce expression of the recombinant gene.

After culturing at 20 °C for 16 to 18 hours, the cultured E. *coli* was centrifuged at 4,500 rpm for 10 minutes to recover the cell precipitate, followed by suspending the precipitate in 5 ml of a disruption solution (10 mM Tris-HCl buffer, pH 7.5, 10 mM EDTA), and then crushing the same using an ultrasonic crusher (Branson Ultrasonics Corp., Danbury, CT, USA). After crushing, centrifugation was performed at 13,000 rpm for 10 minutes to separate the supernatant and insoluble aggregates. The separated supernatant was used for subsequent experiments.

**3. Purification of protein**

The supernatant obtained in Example 2 above was purified through a three-step process. First, 1) $Ni^{2+}$-NTA affinity chromatography using the binding of histidine and nickel fused to the recombinant protein was conducted, 2) the recombinant protein was concentrated and a fluorescent material was adhered through buffer exchange, and 3) finally, in order to separate only the self-assembled protein nanoparticles to which the fluorescent material is adhered, sucrose gradient ultracentrifugation was performed. Details of each step are as follows.

1) $Ni^{2+}$-NTA affinity chromatography

In order to purify a recombinant protein, *E. coli* cultured in the same manner as specified above was recovered, cell pellets thereof were resuspended in 5 mL lysis buffer (pH 8.0, 50 mM sodium phosphate, 300 mM NaCl, 20 mM imidazole), and cells were crushed using an ultrasonicator. The lysed cell solution was centrifuged at 13,000 rpm for 10 minutes to separate only the supernatant, and then each recombinant protein was separated using a $Ni^{2+}$-NTA column (Qiagen, Hilden, Germany) (washing buffer: pH 8.0, 50 mM sodium phosphate, 300 mM NaCl, 80 mM imidazole / elution buffer: pH 8.0, 50 mM sodium phosphate, 300 mM NaCl, 200 mM imidazole).

2) Sucrose gradient ultrafast centrifugation

After adding sucrose by concentration to PBS (2.7 mM KCl, 137 mM NaCl, 2 mM $KH_2PO_4$, 10 mM $Na_2HPO_4$, pH 7.4) buffer, thus to prepare each of 40%, 35%, 30%, 25% and 20% sucrose containing solutions, 2 ml of each sucrose solution at the concentration (45-20%) was placed in an ultracentrifugation tube (ultraclear 13.2 ml tube, Beckman) in sequential order from the highest concentration. Then, after filling 1 ml of the recombinant protein solution present in the prepared self-assembly buffer, ultrafast centrifugation was performed at 35,000 rpm and 4 °C for 16 hours (Ultracentrifuge L-90k, Beckman). After centrifugation, the upper layer (20-25% sucrose solution part) was carefully replaced with PBS buffer using a pipette and an ultracentrifugal filter as specified in the above item 2).

**4. Identification of water-soluble fractions of proteins**

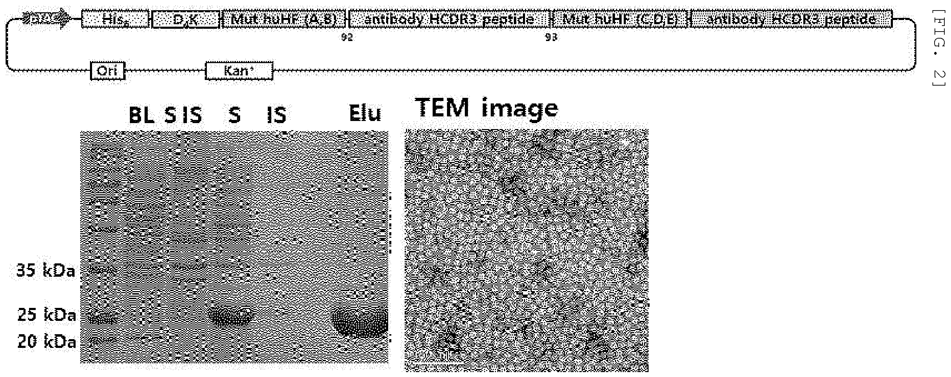

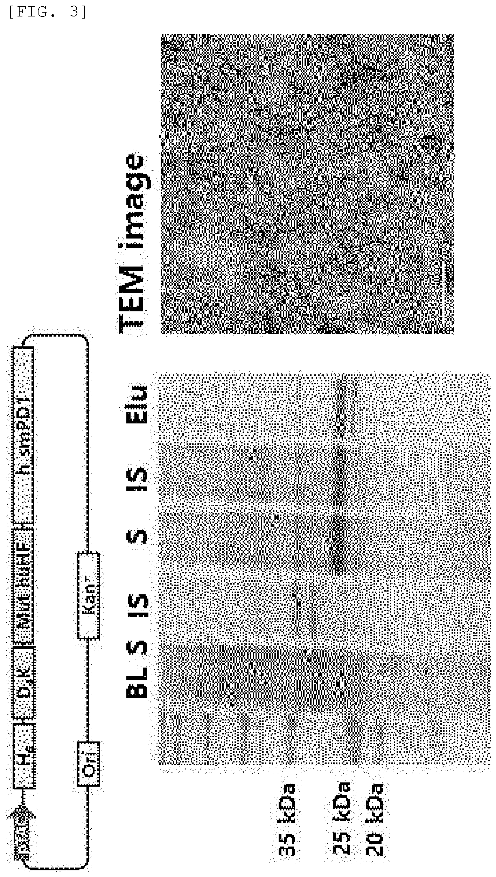

BL21 competent cells were transformed with various expression vectors based on pCM (Tac promoter). A single colony was inoculated to LB liquid medium (50 mL) supplemented with 100 mg/L of kanamycin, and cultured at 37 °C and 130 rpm in a shaking incubator. When the turbidity/optical density at 600 nm reached 0.5, the expression of a target protein was induced through administration of 1 mM IPTG. After 12-16 hours of incubation at 20 °C, the cells in the culture medium were spun-down through centrifugation (13,000 rpm, 10 minutes), and cell pellets were collected and resuspended in 10 mM Tris-Hcl (pH 7.4) buffer. The resuspended cells were ruptured using a Branson Sonifier (Branson Ultrasonics Corp., Danbury, CT). After sonication, the supernatant containing soluble protein and aggregates containing insoluble protein were separated by centrifugation (13,000 rpm, 10 min). The separated soluble and insoluble protein fractions were identified through SDS-PAGE analysis (FIGS. 2 and 3).

**5. Verification of protein assembly**

For structural analysis of the purified recombinant protein nanoparticles of each protein prepared in Example 3, the recombinant protein was photographed by a transmission electron microscope (TEM). First, unstained and purified protein samples were placed on carbon-coated copper electron microscope grids and then natural dried. To obtain stained images of proteins, electron microscope grids containing naturally dried samples were incubated with 2% (w/v) aqueous uranyl acetate solution at room temperature for 10 minutes and washed 3-4 times with distilled water. As a result of observing the protein image using a Philips Technai 120 kV electron microscope, it was confirmed that each particle forms spherical nanoparticles (FIG. 2, FIG. 3).

**6. Measurement of binding force of protein to antigen**

The native form of the proteins prepared in the above examples and the binding force of the Mutant (huHF_mutant1-dual and huHF_mutant2-dual) to human transferrin receptor were compared.

The binding force A of the prepared protein to the antigen was measured according to the following method.

First, human transferrin receptor-Fc tag standard was attached to a high-binding 96 well plate by 100 μl at 2 μg/ml overnight at 4 °C.

Then, it was washed 3 times with 200 ul of PBST (PBS+Tween0.05%). SuperBlcok™ (PBS) Blocking Buffer (thermo cat#37515) was subjected to a blocking process by 100 μl at room temperature for 1 hour.

Then, it was washed 3 times with 200 ul of PBST (PBS+Tween0.05%). The proteins prepared in the above examples were treated overnight at 4 °C by 100 μl for each concentration section.

Then, it was washed 3 times with 200 ul of PBST (PBS+Tween0.05%). An antibody (mouse anti-His tag antibody, Hisprobe) that specifically binds to the hexa-His tag was diluted 1/1000 in PBST, and the diluted solution was treated by 100 μl at room temperature for 1 hour.

Subsequently, it was washed 3 times with 200 ul of PBST (PBS+Tween0.05%). An antibody that specifically binds to a mouse antibody (goat anti-mouse igG antibody-HRP) was diluted 1/1000 in PBST, and the diluted solution was treated by 100 μl at room temperature for 1 hour.

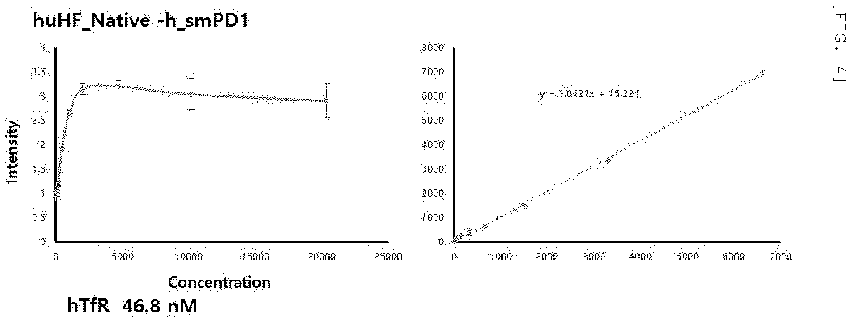

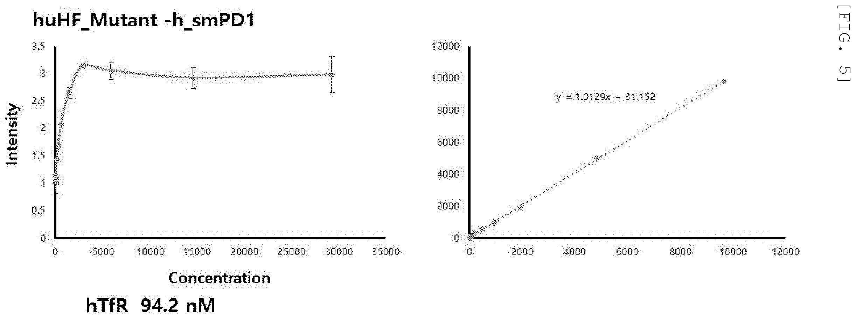

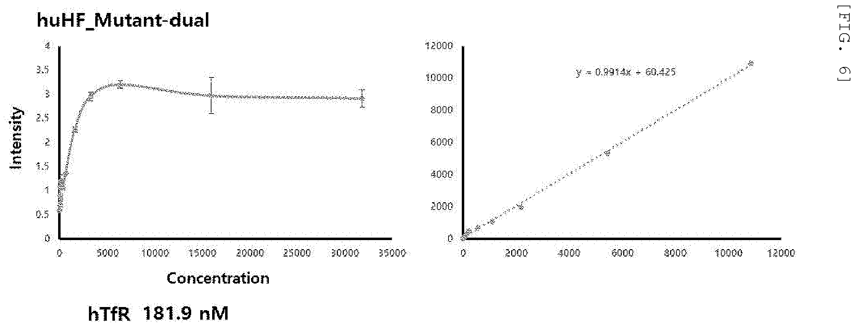

Then, it was washed 3 times with 200 ul of PBST (PBS+Tween0.05%). TMB solution expressing coloration by reacting with HRP was treated by 100 μl at room temperature for 15 minutes. After 50 μl of 1M $H_2SO_4$ solution was treated, optical density was measured with TECAN at a wavelength of 450 nm. This was calculated using the Langmuir equation (Table 4, FIGS. 4 to 6).

[TABLE4]

| Division | | Target ability |
|---|---|---|
| Antibody | Human | 889-2223 |
| | Mouse | 985-2463 |
| huHF_Mutant1-dual | Human | 44-89 |
| | Mouse | 46-93 |

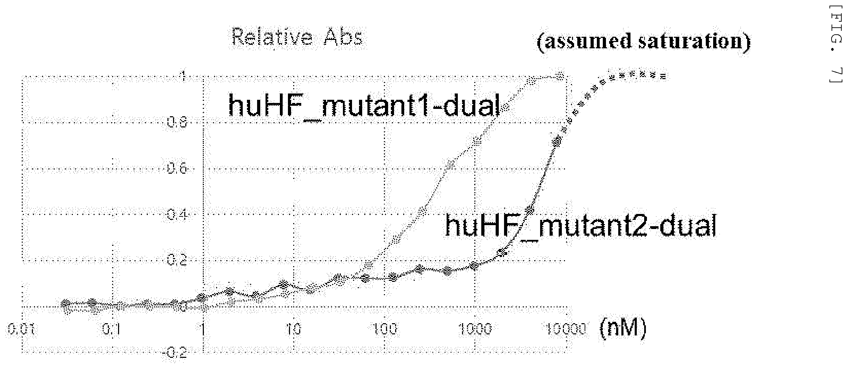

FIG. 7 illustrates results of evaluation/comparison of the binding force of huHF_mutant1-dual and huHF_mutant2-dual to hTfR. Referring to this drawing, in the case of huHF_mutant2-dual, even when the concentration of the protein was increased to 10000 nM, it was not saturated, thereby demonstrating that the binding force to hTfR was further reduced. When the expected saturation point is selected and the binding force Kd value is calculated, it becomes about 4400 nM.

**8. Assessment of binding ability between NK cells and cancer cells according to huHF_mutant-dual treatment**

A549, a human lung cancer cell line, was labeled with CFSE as a fluorescent material. $10^6$ cells were reacted with CFSE at a concentration of 5 μM in an incubator at 37 °C for 20 minutes, washed with PBS, and cultured for one day by diluting $10^5$ cells in 2 ml culture media on a 2 ml cell plate.

Thereafter, $10^5$ NK cells were reacted with the A549 cell line stained with CFSE.

At this time, 10 μl of PBS was used as a control, while 10 μl of 3 μM huHF_mutant-dual (dICB, dual immune checkpoint blocker) labeled with Cy5.5 fluorescent dye was added as an experimental group.

After incubation for 10 minutes in an incubator, NK cells were labeled using an NK 1.1 antibody-PE antibody.

Samples were fixed with 4% formaldehyde, and fluorescence images were identified by fluorescence microscopy.

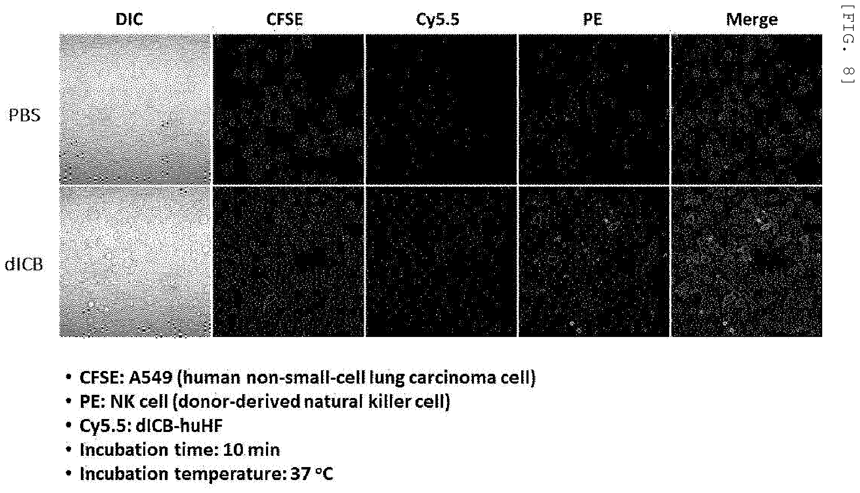

It was confirmed that more NK cells were adhered to A549 cells in the fluorescence image of the experimental group (huHF_mutant-dual) compared to the control (PBS) (FIG. 8) .

**9. Assessment of binding ability between NK cells and cancer cells and cancer cell killing ability of NK cells according to huHF_mutant-dual treatment**

A549, a human lung cancer cell line, was labeled with CFSE as a fluorescent material. $10^6$ cells were reacted with CFSE at a concentration of 5 μM in an incubator at 37 °C for 20 minutes, washed with PBS, and cultured for one day by diluting $10^4$ cells in 200 μl culture media on a 96 well plate.

Thereafter, $10^4$ to $10^5$ NK cells labeled with DAPI were reacted with the A549 cell line stained with CFSE depending on the conditions.

At this time, 10 μl of PBS, 10 μl of 3 μM Abs (Tecentriq+mAb-mTIGIT (Bio X Cell, #BE0274) coadministration group) (each 3 μM, total 6 μM), and 10 μl of 3 μM huHF_mutant-dual were added.

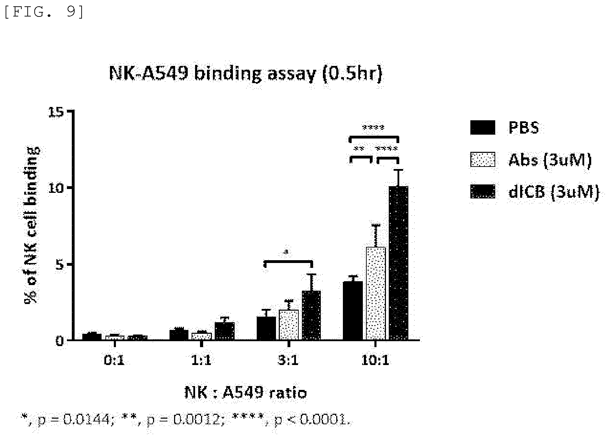

For binding measurement, the reactant solution was reacted in an incubator for 30 minutes, and then, a ratio of cells simultaneously exhibiting CFSE fluorescence and DAPI fluorescence simultaneously was analyzed with FACS equipment (FIG. 9). Referring to this, it could be confirmed that the binding ability of NK cells to A549 cells is increased during huHF_mutant-dual treatment.

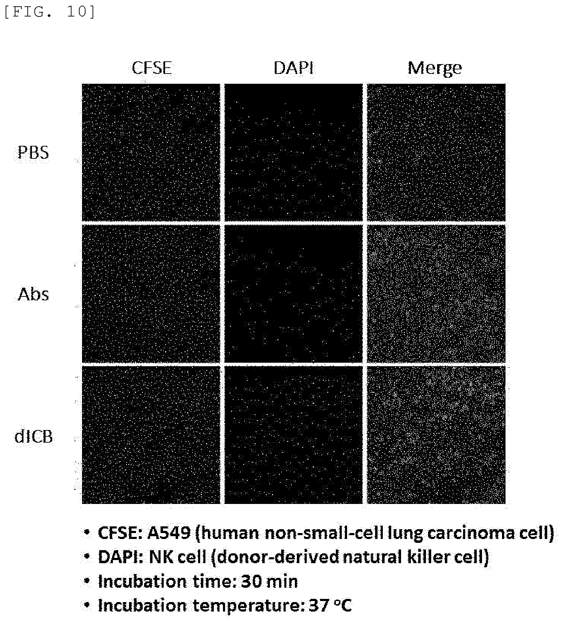

Fluorescence microscopy was used to confirm the fluorescence image (FIG. 10). NK cells were labeled with the NK 1.1 antibody-PE antibody.

For toxicity measurement, the reactant solution was reacted in an incubator for 48 hours, and then, toxic ability according to the sample was verified with FACS equipment. A degree of cell death was analyzed with 7-AAD.

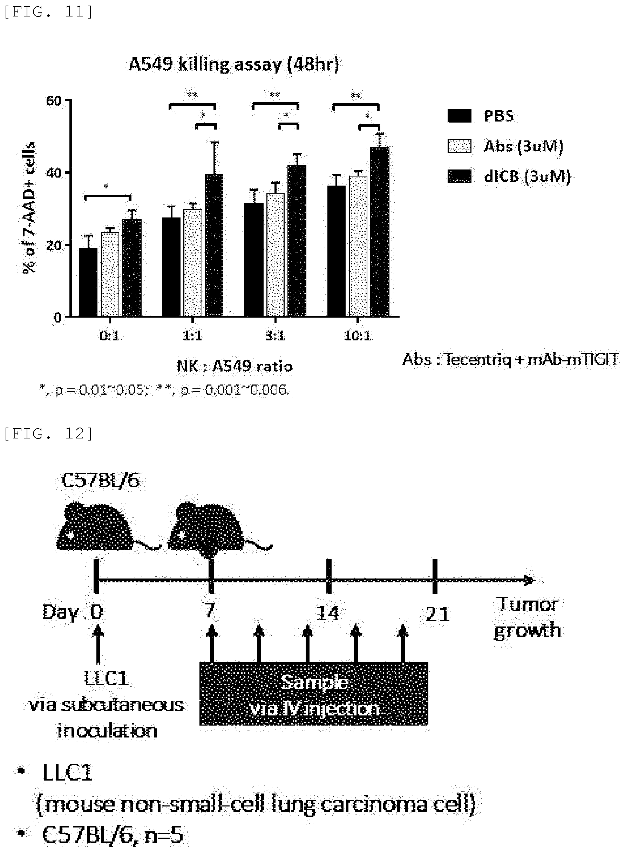

By analyzing a ratio of cells simultaneously exhibiting CFSE fluorescence and 7-AAD fluorescence, a ratio of NK cells to cancer cells and the toxic ability of NK cells according to the sample were analyzed (FIG. 11). Referring to this, it could be confirmed that A549 cell killing ability of NK cells is increased during huHF _mutant-dual treatment.

**10. Assessment of cancer cell growth inhibitory ability**

For each of the following sample groups (PBS, Tecentriq, Tecentriq+mAb-mTIGIT and huHF_mutant-dual), the efficacy of inhibiting normal cancer cells against LLC1, a mouse lung cancer cell line, was verified by 5 mice each. Tecentriq is a monoclonal antibody that binds to PD-1 or PD-L1 used for the treatment of lung cancer, etc., while mAb-mTIGIT is a monoclonal antibody that binds to murine TIGIT.

A tumor model was formed by subcutaneously transplanting LLC1 into C57BL/6 by $5 \times 10^5$ each.

After 7 days, the tumor size was measured and classified for each individual, and the same size was adjusted for each group.

From the 7th day, the sample was intravenously injected through the caudal vein every 3 days, and a tumor size was measured for each individual to compare the tumor growth inhibition efficacy (FIG. 12).

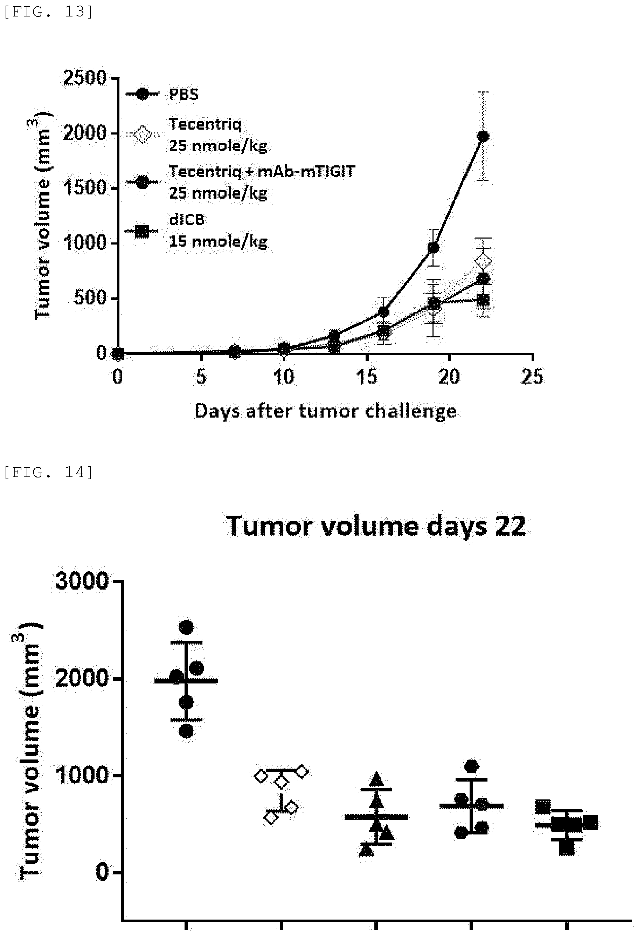

FIG. 13 shows the tumor size by date in each experimental group, in particular, FIG. 14 shows the tumor size on day 22 in each experimental group. Referring to these drawings, in the case of huHF_mutant-dual, it could be confirmed that effects are excellent because the tumor size is the smallest despite treatment with a smaller dose.

**11. Cell avidity assessment**

The avidity of huHF_mutant-dual and mut-huHF against human lung cancer cell line A549 was analyzed.

First, target cells (immune cells, cancer cells) were cultured and dispensed.

($1 \times 10^5$ pieces/10 μl, 1 FACS column (5 ml polystyrene round-bottom tube, Falcon (352052))).

Then, the sample (drug, antibody (anti-mouse PD-L1 antibody (BE0101), anti-mouse TIGIT antibody (BE0274), anti-human PD-L1 (BE0285))) was treated with 100 μl/FACS column, and the control was treated with 100 ul of FACS buffer (0.1% BSA + 0.01% sodium azide in PBS) (3hr, room temperature).

After putting 1 ml of each FACS buffer into the FACS column, centrifugation was performed at 1,700 rpm for 5 minutes, and the process of removing the supernatant was repeated 3 times.

Thereafter, fluorescence antibody (anti-his tag antibody-PE (SC-8036PE), anti-IgG antibody-PE (PE goat F(ab')2 anti-rat (IgG) secondary antibody (ab7010), PE F(ab')2-goat anti-mouse IgG (H+L) secondary antibody (12-4010-82))) was used for treatment (2 hr, room temperature).

After putting 1 ml of each FACS buffer into the FACS column, centrifugation was performed at 1,700 rpm for 5 minutes, and the process of removing the supernatant was repeated 3 times.

Then, after putting 100 μl of FACS buffer in each sample FACS column, it was treated with 2 μl of 7-AAD (BD Pharmigen (559925)) for 5 minutes.

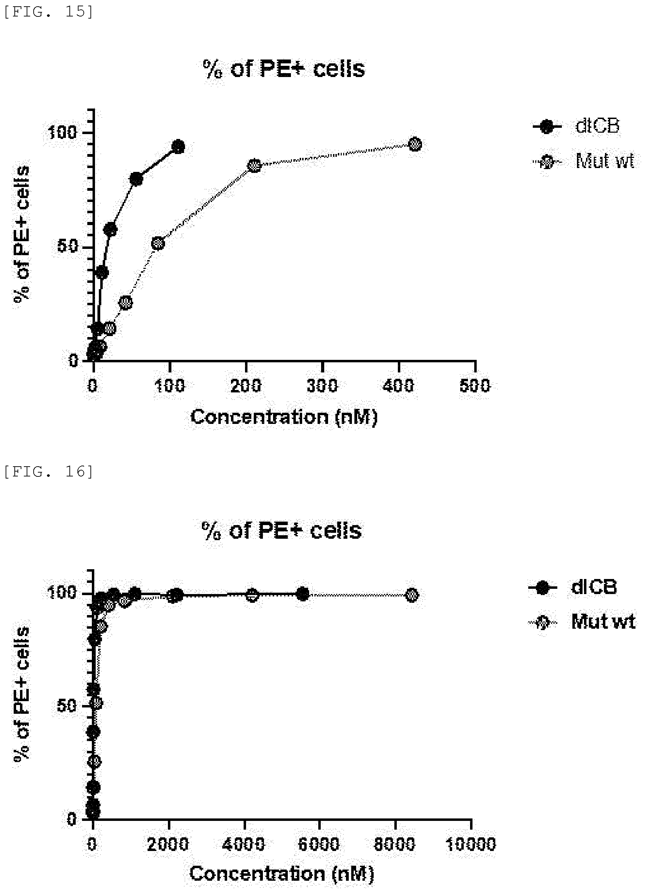

Analysis was performed by means of BD Biosciences and FACS equipment using the ratio of target cells showing PE fluorescence among live cells in each FACS column (FIGS. 15-17).

Through this, it was verified that huHF_mutant-dual had a greater number of samples bound per cell and superior number of bound cells.

**12. Assessment of tumor growth inhibitory ability**

For each of the following sample groups (PBS, Ab-1, huHF_mutant-dual and huHF_mutant-dual+Vac), the efficacy of inhibiting normal cancer cells was verified against LLC1, a mouse lung cancer cell line, by 5 mice each. Ab-1 used herein was mouse anti-PD-L1 antibody (Bio X Cell, #BE0101), and LLC-1 neo-antigen vaccine (SEQ ID NO: 10) was used as the vaccine (Vac).

A tumor model was formed by subcutaneously transplanting LLC1 into C57BL/6 by $5 \times 10^5$.

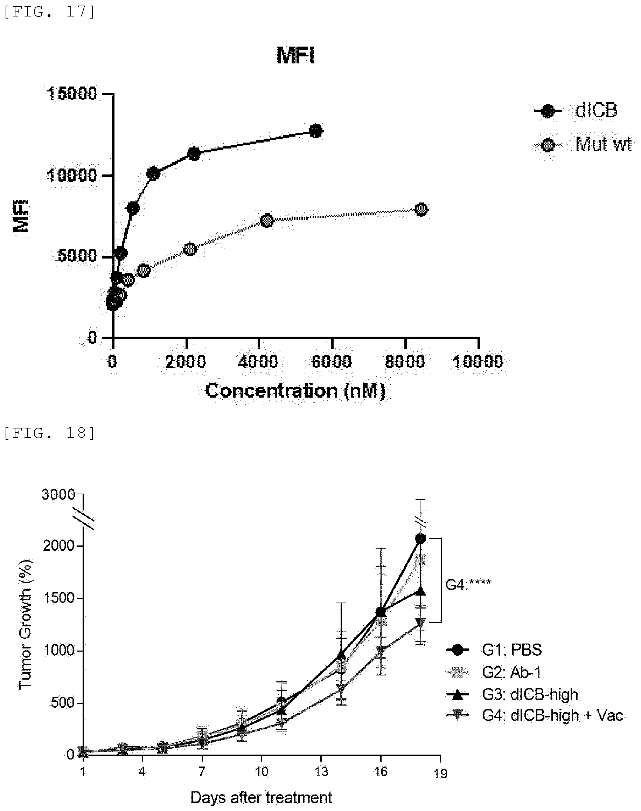

From the 7th day, the sample was intravenously injected through the caudal vein every 3 days (in the case of the G2 group, injected every 6 days), and the tumor size was measured for each individual to compare the tumor growth inhibition efficacy (FIG. 18).

Ab-1 was injected at a concentration of 30 nmol/kg, huHF_mutant-dual was 30 nmol/kg, and Vac was 10 nmol/kg.

Referring to this result, it could be confirmed that the huHF_mutant-dual treatment group has excellent tumor growth inhibitory ability, and exhibits more excellent effects when using in combination with a vaccine.

**Claims**

1. A ferritin protein, comprising a foreign peptide fused to an outer surface thereof, which is mutated so that a binding force to a human transferrin receptor is reduced.

2. The ferritin protein according to claim 1, wherein the foreign peptide is a pharmacologically active peptide.

3. The ferritin protein according to claim 1, wherein the foreign peptide includes a ligand or a fragment thereof, a receptor or a fragment thereof, an antibody or a fragment thereof including an antigen binding region (CDR), which are capable of binding to an immune checkpoint molecule; or a disease antigen epitope.

4. The ferritin protein according to claim 3, wherein the immune checkpoint molecule is any one selected from the group consisting of Her-2/neu, VISTA, 4-1BBL, Galectin-9, Adenosine A2a receptor, CD80, CD86, ICOS, ICOSL, BTLA, OX-40L, CD155, BCL2, MYC, PP2A, BRD1, BRD2, BRD3, BRD4, BRDT, CBP, E2F1, MDM2, MDMX, PPP2CA, PPM1D, STAT3, IDH1, PD1, CTLA4, PD-L1, PD-L2, LAG3, TIM3, TIGIT, BTLA, SLAMF7, 4-1BB, OX-40, ICOS, GITR, ICAM-1, BAFFR, HVEM, LFA-1, LIGHT, NKG2C, SLAMF7, NKp80, LAIR1, 2B4, CD2, CD3, CD16, CD20, CD27, CD28, CD40L, CD48, CD52, EGFR family, AXL, CSF1R, DDR1, DDR2, EPH receptor family, FGFR family, VEGFR family, IGF1R, LTK, PDGFR family, RET, KIT, KRAS, NTRK1 and NTRK2.

5. The ferritin protein according to claim 3, wherein the antigen-binding site is any one selected from the group consisting of HCDR1, HCDR2, HCDR3, LCDR1, LCDR2 and LCDR3.

6. The ferritin protein according to claim 3, wherein the disease antigen epitope is selected from the group consisting of gp100, MART-1, Melna-A, MAGE-A3, MAGE-C2, Mammaglobin-A, proteinsase-3, mucin-1, HPV E6, LMP2, PSMA, GD2, hTERT, PAP, ERG, NA17, ALK, GM3, EPhA2, NA17-A, TRP-1, TRP-2, NY-ESO-1, CEA, CA 125, AFP, Survivin, AH1, ras, G17DT, MUC1, Her-2/neu, E75, p53, PSA, HCG, PRAME, WT1, URLC10, VEGFR1, VEGFR2, E7, Tyrosinase peptide, B16F10, EL4, neoantigens and GV1001.

7. The ferritin protein according to claim 1, wherein the foreign peptide is fused to at least one between adjacent α-helices of the ferritin monomer.

8. The ferritin protein according to claim 1, wherein the foreign peptide is fused to N-terminus or C-terminus of the ferritin monomer.

9. The ferritin protein according to claim 1, wherein the foreign peptide is fused to A-B loop, B-C loop, C-D loop or D-E loop of the ferritin monomer.

10. The ferritin protein according to claim 1, wherein the foreign peptide is fused between N-terminus and A helix or between E helix and C-terminus of the ferritin monomer.

11. The ferritin protein according to claim 1, wherein the mutated ferritin protein is **characterized in that** amino acid No. 15, 16, 23, 82, 84, 117, 120 or 124 in the sequence of SEQ ID NO: 1 is substituted with alanine, glycine, valine or leucine.

13

**12.** The ferritin protein according to claim 1, wherein the binding force (K) to the transferrin receptor satisfies Equation 1 below:

[Equation 1]

$$K \geq 10 \text{ nM}$$

(in Equation 1, K is [P][T]/[PT], wherein [P] represents a concentration of ferritin protein in an equilibrium state of a binding reaction between the ferritin protein and the transferrin receptor, [T] represents a concentration of transferrin receptor in the equilibrium state, and [PT] represents a concentration of a complex of the ferritin protein and the transferrin receptor in the equilibrium state).

**13.** The ferritin protein according to claim 12, wherein the transferring receptor is a human transferring receptor.

**14.** The ferritin protein according to claim 1, wherein the ferritin is a human ferritin heavy chain.

[FIG. 1]

Mut huHF (A,B) | antibody HCDR3 peptide | Mut huHF (C,D,E) | antibody HCDR3 peptide

His₆ | D,K

92        93

Ori    Kan⁺

BL S IS   S   IS      Elu      TEM image

35 kDa

25 kDa
20 kDa

[FIG. 2]

EP 4 335 867 A1

[FIG. 3]

[FIG. 4]

[FIG. 5]

[FIG. 6]

huHF_Mutant-dual

hTfR 181.9 nM

y = 0.9914x + 60.425

[FIG. 7]

Relative Abs     (assumed saturation)

huHF_mutant1-dual

huHF_mutant2-dual

0.01    0.1    1    10    100    1000    10000   (nM)

[FIG. 8]

EP 4 335 867 A1

| | DIC | CFSE | Cy5.5 | PE | Merge |
|---|---|---|---|---|---|
| PBS | | | | | |
| dICB | | | | | |

- **CFSE: A549 (human non-small-cell lung carcinoma cell)**
- **PE: NK cell (donor-derived natural killer cell)**
- **Cy5.5: dICB-huHF**
- **Incubation time: 10 min**
- **Incubation temperature: 37 °C**

[FIG. 9]

**NK-A549 binding assay (0.5hr)**

*, p = 0.0144; **, p = 0.0012; ****, p < 0.0001.

[FIG. 10]

|  | CFSE | DAPI | Merge |

- **CFSE: A549 (human non-small-cell lung carcinoma cell)**
- **DAPI: NK cell (donor-derived natural killer cell)**
- **Incubation time: 30 min**
- **Incubation temperature: 37 °C**

[FIG. 11]

A549 killing assay (48hr)

[FIG. 12]

- LLC1
(mouse non-small-cell lung carcinoma cell)
- C57BL/6, n=5

[FIG. 13]

[FIG. 14]

## Tumor volume days 22

[FIG. 15]

% of PE+ cells

[FIG. 16]

% of PE+ cells

[FIG. 17]

[FIG. 18]

## INTERNATIONAL SEARCH REPORT

| International application No. |
|---|
| **PCT/KR2022/006356** |

### A. CLASSIFICATION OF SUBJECT MATTER

**C07K 14/47**(2006.01)i; **C12N 15/70**(2006.01)i

According to International Patent Classification (IPC) or to both national classification and IPC

### B. FIELDS SEARCHED

Minimum documentation searched (classification system followed by classification symbols)

C07K 14/47(2006.01); A61K 31/69(2006.01); A61K 38/16(2006.01); A61K 9/51(2006.01); C07K 14/79(2006.01); C07K 19/00(2006.01); C12N 15/62(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 페리틴(ferritin), 트랜스페린 수용체(transferrin receptor), 융합(fusion), 돌연변이 (mutation), 암(cancer)

### C. DOCUMENTS CONSIDERED TO BE RELEVANT

| Category* | Citation of document, with indication, where appropriate, of the relevant passages | Relevant to claim No. |
|---|---|---|
| Y | KR 10-2018-0008353 A (KOREA INSTITUTE OF SCIENCE AND TECHNOLOGY) 24 January 2018 (2018-01-24)<br>See claims 1-2, 4-5 and 7-19; paragraphs [0014], [0026], [0036]-[0037] and [0063]; and figures 1a-1b. | 1-14 |
| Y | MONTEMIGLIO, L. C. et al. Cryo-EM structure of the human ferritin–transferrin receptor 1 complex. Nature Communications. 2019, vol. 10, thesis no. 1121, pp. 1-8.<br>See abstract; page 4; figure 3; and supplementary figure 4. | 1-14 |
| Y | KR 10-2017-0027683 A (KYUNGPOOK NATIONAL UNIVERSITY INDUSTRY-ACADEMIC COOPERATION FOUNDATION et al.) 10 March 2017 (2017-03-10)<br>See abstract; claims 1-18; and figures [0062]-[0066] and [0085]-[0103]. | 1-14 |
| Y | KR 10-2017-0047120 A (KOREA UNIVERSITY RESEARCH AND BUSINESS FOUNDATION et al.) 04 May 2017 (2017-05-04)<br>See claims 1-5 and 9-21. | 1-14 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **18 August 2022** | **18 August 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2022/006356** |

**C.    DOCUMENTS CONSIDERED TO BE RELEVANT**

| Category* | Citation of document, with indication, where appropriate, of the relevant passages | Relevant to claim No. |
| --- | --- | --- |
| Y | KR 10-2015-0088597 A (UNIST ACADEMY-INDUSTRY RESEARCH CORPORATION) 03 August 2015 (2015-08-03)<br>See claims 1-20; and paragraph [0021]. | 1-14 |

Form PCT/ISA/210 (second sheet) (July 2019)

## INTERNATIONAL SEARCH REPORT

| International application No. |
| --- |
| **PCT/KR2022/006356** |

**Box No. I**      **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑  forming part of the international application as filed:

   ☑  in the form of an Annex C/ST.25 text file.

   ☐  on paper or in the form of an image file.

   b.  ☐  furnished together with the international application under PCT Rule 13*ter*.1(a) for the purposes of international search only in the form of an Annex C/ST.25 text file.

   c.  ☐  furnished subsequent to the international filing date for the purposes of international search only:

   ☐  in the form of an Annex C/ST.25 text file (Rule 13*ter*.1(a)).

   ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
Information on patent family members

International application No.

**PCT/KR2022/006356**

| Patent document cited in search report | | | Publication date (day/month/year) | Patent family member(s) | | | Publication date (day/month/year) |
|---|---|---|---|---|---|---|---|
| KR | 10-2018-0008353 | A | 24 January 2018 | CA | 3040440 | A1 | 18 January 2018 |
| | | | | CN | 109803642 | A | 24 May 2019 |
| | | | | EP | 3501509 | A1 | 26 June 2019 |
| | | | | EP | 3501509 | A4 | 08 July 2020 |
| | | | | KR | 10-1953617 | B1 | 23 May 2019 |
| | | | | US | 10905774 | B2 | 02 February 2021 |
| | | | | US | 2019-0216947 | A1 | 18 July 2019 |
| | | | | WO | 2018-012952 | A1 | 18 January 2018 |
| KR | 10-2017-0027683 | A | 10 March 2017 | KR | 10-1888675 | B1 | 14 August 2018 |
| | | | | US | 10781238 | B2 | 22 September 2020 |
| | | | | US | 2018-0291072 | A1 | 11 October 2018 |
| | | | | WO | 2017-039382 | A1 | 09 March 2017 |
| KR | 10-2017-0047120 | A | 04 May 2017 | KR | 10-2389169 | B1 | 21 April 2022 |
| | | | | US | 10206987 | B2 | 19 February 2019 |
| | | | | US | 2017-0112910 | A1 | 27 April 2017 |
| KR | 10-2015-0088597 | A | 03 August 2015 | KR | 10-1630858 | B1 | 15 June 2016 |

Form PCT/ISA/210 (patent family annex) (July 2019)